# EUROPEAN PATENT APPLICATION

(11) **EP 1 323 436 A1**
(43) Date of publication of application: **02.07.2003**
(21) Application number: 02080404.3
(22) Date of filing: 19.12.2002
(51) Int. Cl.: A61L 15/28, A61L 15/50

(54) **Anti-adhesion barrier comprising carboxymethylcellulose and gellan gum**

(30) Priority: 26.12.2001 KR 2001085142; 28.10.2002 KR 2002065986
(71) Applicant: Amitie Co., Ltd., Chonju, Chonbuk-do, 561-203 (KR)
(72) Inventor: Kim, Dae Sik, Wansan-gu, Jeonju-si,Jeonrabukdo 560-240 (KR); Kim, Do Hoon, Seo-gu, Gwangju, 502-240 (KR); Lee, Jeong Kwon, Nonsan-si, Chungcheongnam-do, 320-900 (KR)
(74) Representative: SERJEANTS

(57) **Abstract**

Anti-adhesion barrier compositions for preventing adhesion between tissues at an surgical lesion comprising sodium carboxymethyl cellulose and gellan gum in a weight ratio of 1 : (0.2 to 5), preferably 1 : (0.8 to 1.25). The compositions may further contain from 0.2 to 5 parts by weight of one or more of interleukine-10, hyaluronate, heparin, nifedipine HCl, phosphatidylcholine, medroxyprogestone acetate, L-arginine, nitric oxide, polyvinylalcohol (PVA), protein inhibitor and dextran per 1 part by weight of the sodium carboxymethyl cellulose and/or from 0.02 to 0.06 parts by weight of an antibiotic substance per 1 part by weight of the sodium carboxymethyl cellulose.

## Description

The invention relates to anti-adhesion barrier compositions which suppress bleeding at surgical lesions (haemostatic effect) and prevent a mutual adhesion of tissues at surgical lesions (anti-adhesion effect).

Bleeding during or after surgical operations may cause tissue adhesions and scarring. Particularly when organs bind to tissues or other organs, pain and a reduction of organ function can result and may necessitate fresh surgical intervention for correction. For example, adhesions are reported to be provoked in 70 to 95% of abdominal operations (Eur. J. Surg., 1997, Suppl., 577, 10-16, 24-31, 32-39) with a substantial risk of adhesion between the abdominal cavity wall and the intestine.

Much research has been carried out to develop anti-adhesion barriers which can be degraded in a body, have low toxicity and prevent mutual adhesions of organs for a short time or a long time. Further desirable characteristics of an effective anti-adhesion barrier include good adhesiveness, applicability, affinity to tissues and anti-bacterial properties.

Recently, various studies have sought to develop anti-adhesion barriers which can block a contact onto a lesion site after an operation. In this anti-adhesion barrier, bioadsorptive polymer having a carboxyl terminal group in a high molecular weight is utilized and can be hydrated within a body. Then, the lesion site is separated from adjacent tissues during a treatment period for the lesion so that the caused adhesion is prevented. Moreover, after the treatment is completed, the anti-adhesion barrier might be absorbed and removed naturally, which does not affect onto normal tissues.

US 4141973 has disclosed that biodegradable polymer such as hyaluronic acid (HA) could be used for this method. Unfortunately, HA cannot be applied to an anti-adhesion barrier in practice since it is degraded and absorbed into a body at a relatively high speed.

In the meantime, sodium carboxymethyl cellulose (NaCMC) has been reported as a substance for an anti-adhesion agent in various cases (Fertil. Steril., 1984, June, Gynecol., 1986, 155: 3, 667-670). However, it is known that sodium carboxymethyl cellulose is difficult to be applied to an anti-adhesion barrier since it is absorbed or removed too fast into a body and thus cannot have a preventive effect upon the adhesion.

As demonstrated above, in order to solve the problems of the prior art, there are many trials for effects upon adhesion which is difficult to be manifested due to excessive rapidity of degradation or removal in material of anti-adhesion barriers. Concretely, the method for forming a connection between biodegradable polymers has been illustrated. Precisely, EP 507604 has demonstrated that polysaccharides having a carboxyl terminus were bonded with polyvalent metal ions, which reduces the solubility so as to be utilized as an anti-adhesive barrier. In addition, US 5318780 has illustrated that the anti-adhesive barrier was prepared by using in situ gelatin method.

However, these methods are concluded not to be suitable for an anti-adhesive barrier since they reduce tissue adhesive strength and complicate the process for an operation although they are advantageous to make films within a body by using membrane forming polymer (hydroxypropyl methyl cellulose, HPMC).

On the other hand, US 5017229, US 5527893, US 5760200 and others have disclosed that a kind of polysaccharide such as hyaluronic acid (HA) and carboxymethyl cellulose (CMC) should be utilized to prevent an adhesion. These methods also have some problems in the procedure for removing biologically toxic material generated in the process for the preparation. Hydration is proceeded to make gelatin induced at a high speed, which causes some difficulties in the treatment and the operation, namely preventing a reoperation after separating them again (Surg. Clin. Nor. Am., 1997, 77: 3, 671-688).

General adhesion of hydrophilic biopolymer against body tissues could be affected most by hydration but polymers having a carboxyl terminus bonding with tissues are suitable for this use. Moreover, in case of gel type the adhesiveness of polymer itself is more important factor since already saturated with moisture.

Recently, several operational techniques have been developed and advantages preventing adhesion are acquired additionally (Hepato-Gastroenterol., 1991, 38, 283). This trend for developing anti-adhesion barriers meet changes of these operational techniques and continues to develop anti-adhesion barriers in a solution or a gel type, although solution type, gel type and film type are known to types of anti-adhesion barrier (The Adhesion Prevention Opportunity Report from MDI, 1998).

The gel type anti-adhesion barrier attracts attention recently since it can prevent an adhesion effectively with treating lesion sites in a small amount. Especially, pharmaceutics which can be applied in a gel type restrictively to spine operation, tendon operation and the like have been attempted (US 5605938). Presently, commercially available phosphated saline manufactured by Gliatech Inc. and a gel type anti-adhesion barrier consisting in porcine and polyglycan ester (trademark: ADCON-L) have been utilized widely.

Unfortunately, typical anti-adhesion barriers described above are still restricted in their efficacies such as adhesiveness, affinity, anti-inflammation, anti-bacterial property and regeneration in spite of achievements in anti-adhesiveness and haemostatic function.

The invention provides an anti-adhesion barrier composition comprising sodium carboxymethyl cellulose and gellan gum in a weight ratio of 1 : (0.2 to 5).

Sodium carboxymethyl cellulose (NaCMC) has a regenerative function in lesion sites, haemostatic properties, compatibility for a human body, and safety. The gellan gum controls the absorption of sodium carboxymethyl cellulose into a human body, sustains a gel shape formed in a body and enhances lubrication. The weight ratio of NaCMC to gellan gum is preferably 1 : (0.8 to 1.25). Most preferably, the ratios are almost the same.

Precisely. sodium carboxymethyl cellulose plays a role as a matrix to form gel having an effect for preventing an adhesion in the present invention. Gellan gum plays a role to situate body tissues onto a right position by conferring a lubricant property on gel as well as to sustain a gel shape during a required period by preventing a rapid degradation and absorption of NaCMC.

In the components applied to the composition of the present invention, the molecular weight is not considered as an important factor conventionally, but preferably in case of NaCMC, the molecular weight is about 100,000 to 1,000,000 Da and in case of gellan gum, about 200,000 to 1,000,000 Da. Besides, the molecular weight and the composition of each component can be controlled properly, depending upon a lesion site and a purpose of use. For example, while administered onto sites having many trauma and bleeding, the molecular weight and the composition of NaCMC is preferable to become higher relatively and while administered onto internal organs, the molecular weight and the composition of NaCMC is preferable to be lower relatively.

Neither the molecular weight of the NaCMC nor the amount of the NaCMC in the composition should be too high, as this is likely to block the recovery of organs after the operation. When the molecular weight of gellan gum is too high, or the amount of gellan gum in the composition is too high, tissues can be prevented from returning to their previous position after an operation and recovery of the operative sites can be inhibited since the gel is sustained for longer than necessary. On the other hand, if the molecular weight or the composition of components in the anti-adhesion barriers too low, the efficacy of the anti-adhesion barrier is correspondingly lowered.

Especially, if the molecular weight or the composition of NaCMC is too low, some problems that the prevention of adhesion, haemostatic efficacies and the like would be decreased can be caused.

Concretely, the gel type of anti-adhesion barrier illustrated below can be utilized after the operation of peritoneal cavity organs. At this moment, the concentrations (compositions) of NaCMC and gellan gum are preferable to be adjusted to 5.0 weight % respectively (by using distilled water or small amount of additives), in which the anti-adhesion barrier play a role to lubricate and then to improve the return to original position. The adequate viscosity of the anti-adhesion barrier confers the effectiveness for preventing an adhesion after an operation. In the meantime, in order to enhance the efficacy of anti-adhesion, the anti-adhesion barrier of the invention may further comprise various kinds of natural substances which are known to be non-toxic to a human body, in particular one or more of interleukine-10, hyaluronate, heparin, nifedipine HCl, phosphatidylcholine, medroxyprogestone acetate, L-arginine, nitric oxide, polyvinylalcohol (PVA), protein inhibitor and dextran in a weight ratio of 1 : (0.2 ∼ 5) against sodium carboxymethyl cellulose. At this moment, the protein inhibitor can be selected among tumour necrosis factor (TNF), INF, IL-10, IL-13, IL-1, interferon and the like.

Furthermore, in order to sterilize microbes infected in the process of operation, the anti-adhesion barrier of the present invention can contain antibiotics additionally in a weight ratio of 1 : (0.02 ∼ 0.06) against sodium carboxymethyl cellulose. Suitable antibiotics include penicillins, cephalosporins, aminoglycosides, tetracyclins, chloroamphenicols, quinines, polypeptide system antibiotics and the like. These antibiotics can be added in adequate compositions and processes according to respective characteristics and activities.

The anti-adhesion barrier prepared in the present invention can have various forms and preferably, a foam type, powder type, gel type or the like.

The foam type of anti-adhesion barrier is prepared by a process, comprising steps as follows: (1) making a mixed solution consisting in the above mentioned components and an adequate amount of water; (2) injecting the mixed solution into a container having a suitable shape and size; (3) freezing rapidly by submersing into liquid nitrogen; and (4) lyophilizing under a reduced pressure at less than -55°C for more than 48 hours. As a result, the foam type of anti-adhesion barrier can be prepared to have various shapes and sizes, in accordance with the shape and size of the container. Depending upon requirements, the mixed solution can be more sterilized at the temperature range of 120 ∼ 150°C for 5 ∼ 20 minutes by autoclaving, before the step (3) freezing rapidly.

The powder type of anti-adhesion barrier is prepared by pulverizing the foam type of the anti-adhesion barrier manufactured above so that a particle radius of from 0.2 to 0.6 mm is attaimed. The gel type of anti-adhesion barrier is prepared by the process comprising: (1) adding 4 to 19 parts by weight of sterilized water to 1 part by weight of the powder type of anti-adhesion barrier manufactured above; and (2) stirring.

In order to prevent the contamination by microbes, the above anti-adhesion barrier is preferably prepared under a sterilized condition. In addition, in case of a foam type and a powder type, the anti-adhesion barrier is more sterilized with oxidized ethylene gas (EtO gas) and preferably, when distilled water and the powder type anti-adhesion barrier are mixed in case of a gel type, adequate kinds and compositions of antibiotics are added. Depending upon cases, before lyophilizing during the process for preparing the powder type anti-adhesion barrier, adequate kinds and compositions of antibiotics can be added into the autoclaved mixed solution.

Each type anti-adhesion barrier of the present invention play the same role, except that the gel type can be applied onto an operation site in a gelatinous state and the foam type and the powder type become gelatinous by absorbing body fluid and secreted blood components after applied to a surgical lesion. Therefore, it can be applied properly for the operation, considering the morphological characteristics.

That is to say, the gel type anti-adhesion barrier can be administered by covering all the surgical lesions as long as it is not flowed downward. The foam type anti-adhesion barrier is convenient to be applied when the space formed at an surgical lesion is defined accurately. For example, it can be utilized in spine or bone operations. Namely, in case that the operated portion is a crevice formed between bones, it can be applied by being modified to a proper shape and inserted. The powder type anti-adhesion barrier is utilized by scattering at surgical lesions such as panniculus adiposus, uterus, heart, organs and the like or at portions that the gel is flowed downward.

The anti-adhesion barrier of the invention has been confirmed to have a preventive effect upon adhesion between cells in *in vitro* experiments and not to affect cells negatively (toxicity). In addition, the anti-adhesion barrier of the invention is remarkable in anti-inflammation, anti-bacterial property, a regenerative function at a lesion site, haemostatic property, has an outstanding compatibility for a human body, and a high lubricant property, adhesion property, affinity and the like and also has a good anti-adhesion between cell tissues and haemostatic effects.

The following Examples illustrate the invention.

### Example 1

### Preparation of anti-adhesion barrier

Anti-adhesion barriers having the compositions set out in Table 1 were prepared as powders according to the method (1) below or as foams according to the method (2) below. The NaCMC and the gellan gum had average molecular weights of 250,000 Da and 500,000 Da respectively.
*(Method 1)* each component was dissolved in 90 ml of water, frozen rapidly by submersing the solution in liquid nitrogen and then lyophilized at below -55°C for more than 48 hours. Afterward, the solid matter was produced and pulverized to a powder.
*(Method 2)* each component was dissolved in 90 ml of water, injected into a mould with a proper shape, frozen rapidly by submersing the solution in liquid nitrogen and then lyophilized at below -55°C for more than 48 hours. Afterward, the foam for an anti-adhesion barrier was produced. Naturally, it can be pulverized to make a powder like an anti-adhesion barrier of (1).
In each of the above processes (1) and (2), a sterilization step may be performed at 120°C for 10 minutes before lyophilizing the solution.

**Table 1**

| | NaCMC (g) | gellan gum (g) | Other ingredients (g) |
|---|---|---|---|
| Comparative Composition | 10.0 | - | - |
| Composition 1 | 5.0 | 5.0 | - |
| Composition 2 | 10.0 | 5.0 | - |
| Composition 3 | 15.0 | 5.0 | - |
| Composition 4 | 5.0 | 5.0 | Interleukin-10 5.0 |
| Composition 5 | 5.0 | 5.0 | Hyaluronate 5.0 |
| Composition 6 | 5.0 | 5.0 | Heparin 5.0 |
| Composition 7 | 5.0 | 5.0 | Nifedipine HCl 5.0 |
| Composition 8 | 5.0 | 5.0 | Phosphatididylcholine 5.0 |
| Composition 9 | 5.0 | 5.0 | Medrexyprogesterone acetate 5.0 |
| Composition 10 | 5.0 | 5.0 | L-arginine 5.0 |
| Composition 11 | 5.0 | 5.0 | Nitric oxide 5.0 |
| Composition 12 | 5.0 | 5.0 | PVA 5.0 |
| Composition 13 | 5.0 | 5.0 | Protein inhibitor 5.0 |
| Composition 14 | 5.0 | 5.0 | Dextran 5.0 |

### Example 2

### Anti-adhesion effects - in vitro experiments

The anti-adhesion effects were examined by using the powder compositions prepared in Example 1 in experiments for preventing diffusion by cell growth. In particular, 10 g of the comparative composition was dissolved in 90 ml of water to prepare a comparative gel composition. 10 g of powder composition 1 was dissolved in 90 ml of water to prepare gel composition 1-1. 20 g of powder composition 1 was dissolved in 90 ml of water to prepare gel composition 1-2. 10 g of each of the powder compositions 2 to 14 was dissolved in 90 ml of water to prepare gel composition 2 to 14. Depending upon requirements, penicillin as an antibiotic was added to reach 0.2 weight %.

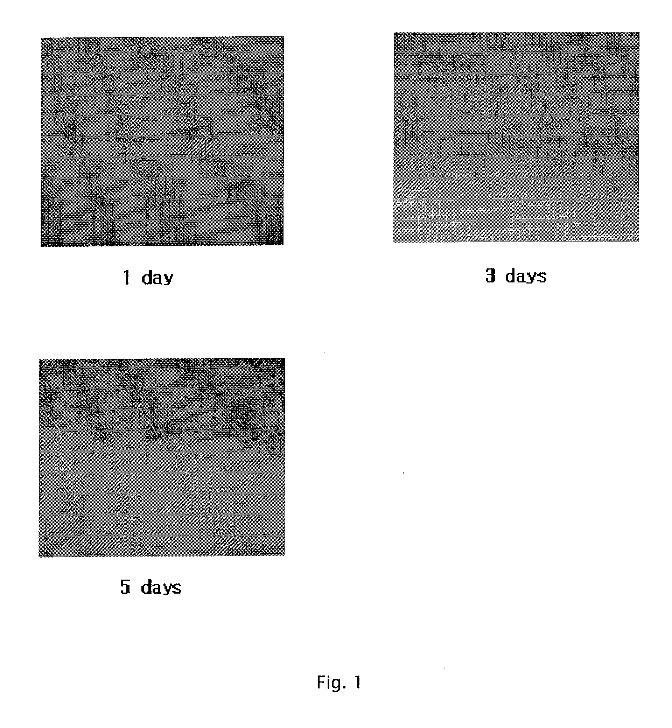

As experimental cells, smooth muscle cell and fibroblast cell were adopted. These cells were poured in 5 x 10⁵ cells and cultivated onto polystylene dish for cell culture with 10 cm of radius and then removed when cells grew to about 0.5 cm of width by using pincet. Afterward, the anti-adhesion barrier prepared in Composition was administered. The treated cells were continued to be cultivated, observed intermittently under a microscope whether the cells can penetrate the covering layer and proliferate and taken to pictures (See FIG. 1). In FIG. 1, each date depicted days lapsed after the anti-adhesion barrier was administered and the upper region of the picture was a cell layer, the lower region of the picture, a covering layer.

As a result, the diffusion of animal cells were blocked in all Examples until 5 days lapsed after cultured. All the experimental results were very similar and thus only one set of the picture that showed the experimental result of Application Example 1-1 were attached in FIG. 1.

As illustrated in FIG. 1, at each step of the observation, a cell domain and a gel application domain were separated definitely with making a border line, which showed that cells could not penetrate a gel application domain.

Therefore, all the anti-adhesion barriers of the present invention were confirmed to have anti-adhesion effects during a short period.

### Example 3

### Toxicity of the Anti-adhesion barriers

(1) The gel compositions prepared in Example 2 were investigated for toxicity against animal cells. The toxicity experiment was conducted as prescribed in ISO 10993-5. L929 cells were cultivated to form a monolayer by using MEM media, treated with trypsin, measured to have 1 x 10 cells/ml concentration and then cultivated again to form a monolayer. Afterward, the anti-adhesion barrier, Adcon™, commercially available from Gliatech Inc., and the compositions prepared in Example 2 were added in 4 ml respectively, cultivated in 5% CO₂ incubator at 37°C and then cell concentrations were estimated in the culture broth after 24 hours and 48 hours. In Toxicity Experiment Example of standard group, the cell concentration at 48 hours lapse was decided to become 100 and the relative concentrations against this value were depicted in Table 2 below.

**Table 2**

| Composition | Relative concentration of cells | |
|---|---|---|
| | After 24 hrs. | After 48 hrs. |
| Comparative Gel Composition | 95 | 100 |
| Adcon™ | 87 | 85 |
| Gel Composition 1-1 | 114 | 108 |
| Gel Composition 4 | 119 | 121 |
| Gel Composition 5 | 125 | 130 |
| Gel Composition 6 | 92 | 95 |
| Gel Composition 7 | 97 | 96 |
| Gel Composition 8 | 101 | 105 |
| Gel Composition 9 | 94 | 101 |
| Gel Composition 10 | 96 | 115 |
| Gel Composition 11 | 90 | 92 |
| Gel Composition 12 | 99 | 101 |
| Gel Composition 13 | 101 | 92 |
| Gel Composition 14 | 103 | 108 |

As illustrated in Table 2, the anti-adhesion barrier compositions of the invention did not show toxicities high enough to inhibit the growth of animal cells. On the other hand, Adcon™ was confirmed to have a considerable toxicity against animal cells.
(2) Gel composition 1-1, as a representative example of the anti-adhesion barrier compositions of the invention, was further investigated by the Korea Testing and Research Institute for Chemical Industry according to the methods set out in Table 3 below. The results are also shown in Table 3.

**Table 3**

| Test | Test Method | Test Result |
|---|---|---|
| Cytotoxicity Test | Notification of Korea Food & Drug Administration ISO 10993-5 | No abnormal result |
| Skin sensitization test | ISO 10993:10 | No abnormal result |
| Acute toxicity test | Notification of Korea Food & Drug Administration | No abnormal result |
| Genetic toxicity test (carcinogenicity test) | USP NF(1998) | No abnormal result |
| Transplantation test | USO NF(1998) | No abnormal result |
| Subacute toxicity test | Notification of Korea Food & Drug Administration | No abnormal result |
| Chronic toxicity test | Notification of Korea Food & Drug Administration | No abnormal result |

In the meantime, substances supplemented additionally in Application Example 4 ∼ 14 (respectively, interleukine-10, hyaluronate, heparin, nifedipine HCl, phosphatidylcholine, medroxyprogestone acetate, L-arginine, nitric oxide, PVA, protein inhibitor and dextran) were known to be compatible to a human body without various levels of toxicity.

Hence, the various compositions of anti-adhesion barriers in the present invention did not cause side effects according to toxicity although administered in a body.

### Example 4

### Anti-adhesion effects - in vivo experiments

### (Experiment 1)

The effects upon anti-adhesion were examined using the gel compositions 1-1, 1-2 and 2 and the comparative gel composition. For these experiments, penicillin as an antibiotic was added at 0.2 weight %.

Mice aged 8 to 24 weeks (weight: 220 to 450g, male) were opened in the dorsal region and thus panniculus adiposus was caused to bleed by effecting small trauma around blood vessels of muscles. Then, the gel type anti-adhesion barrier was applied and the mice were closed.

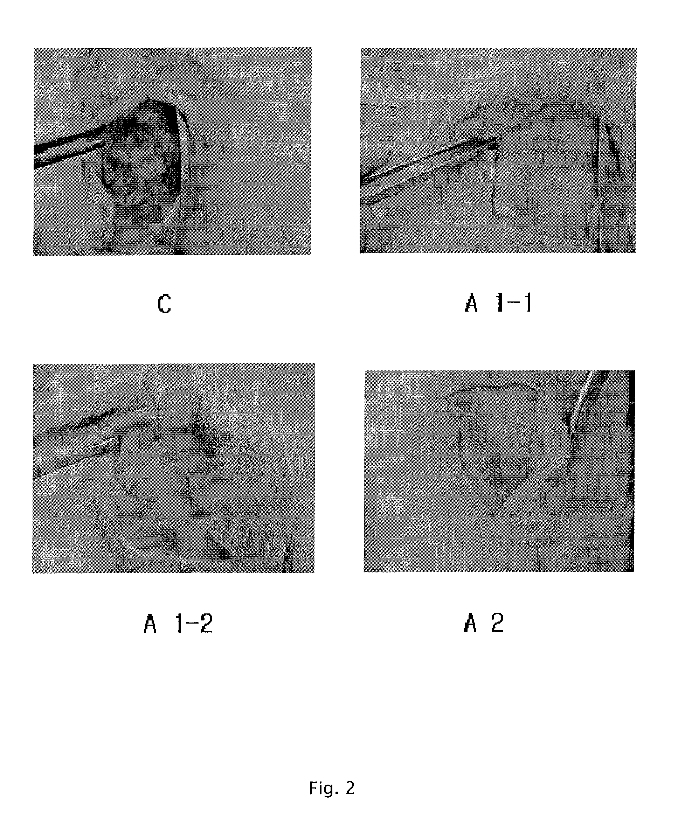

One month after the operation, the operative site was re-opened. Photographs (see Figure 2) were taken to show the anti-adhesion effect and the haemostatic effect. In Figure 2, the photograph designated C shows the effect of the comparative gel composition and the photographs designated A 1-1, A 1-2 and A 2 show the effects of the gel compositions 1-1, 1-2 and 2 respectively. As these photographs show, when the anti-adhesion barriers compositions of the invention comprising NaCMC and gellan gum were administered (A 1-1, A 1-2 and A 2), adhesion at the suture site of the first operation was not found, and neither were bleeding nor inflammation found. On the contrary, when the known anti-adhesion barrier comprising only NaCMC (C) was administered, the adhesion caused at the suture site of the first operation was found and symptoms such as bleeding and inflammation were discovered.

### (Experiment 2)

The effects upon anti-adhesion were examined using the compatative gel composition, gel compositions 1-1, 1-2 and 2 to 14, and Adcon™.

Mice aged 8 weeks (SD rat, 280 to 400g, male) were opened in the dorsal region and muscles around the spine were cut to about 0.5 cm depth and 1 cm length. Then, the gel type anti-adhesion barrier was applied and the mice were closed.

Eight weeks after the operation, the operative site was re-opened. The degrees of adhesion were estimated objectively by performing a double blind method. In detail, observation by the naked eye was performed and the gray holtz grade which was decided through tenacity at the adhesion site were measured. The evaluation is on a scale from 0 to 4, samples in which the adhesion effect was not found being defined as 0 and the sample in which extensive dense adhesion was found being defined as 4. The average Gray Holtz grades given in Table 4 below were from 10 samples in each case except the comparative gel composition and the gel composition 1-1, where the averages are from 20 samples.

**Table 4**

| Composition | Gray Holtz Grade |
|---|---|
| Comparative Gel Composition | 3.65 |
| Adcon™ | 1.4 |
| Gel Composition 1-1 | 0.25 |
| Gel Composition 1-2 | 0.2 |
| Gel Composition 2 | 0.4 |
| Gel Composition 3 | 0.2 |
| Gel Composition 4 | 0.1 |
| Gel Composition 5 | 0.0 |
| Gel Composition 6 | 0.2 |
| Gel Composition 7 | 0.3 |
| Gel Composition 8 | 0.4 |
| Gel Composition 9 | 0.5 |
| Gel Composition 10 | 0.3 |
| Gel Composition 11 | 0.2 |
| Gel Composition 12 | 0.5 |
| Gel Composition 13 | 0.0 |
| Gel Composition 14 | 0.1 |

As illustrated in Table 4, when the anti-adhesion barrier of the invention was applied, adhesion between tissues was rarely observed. On the contrary, when conventional anti-adhesion barriers (the comparative gel composition and Adcon™) were administered, substantial adhesions were found. Especially, the conventional anti-comparative gel composition comprising only NaCMC was shown to have a severe adhesion between tissues.

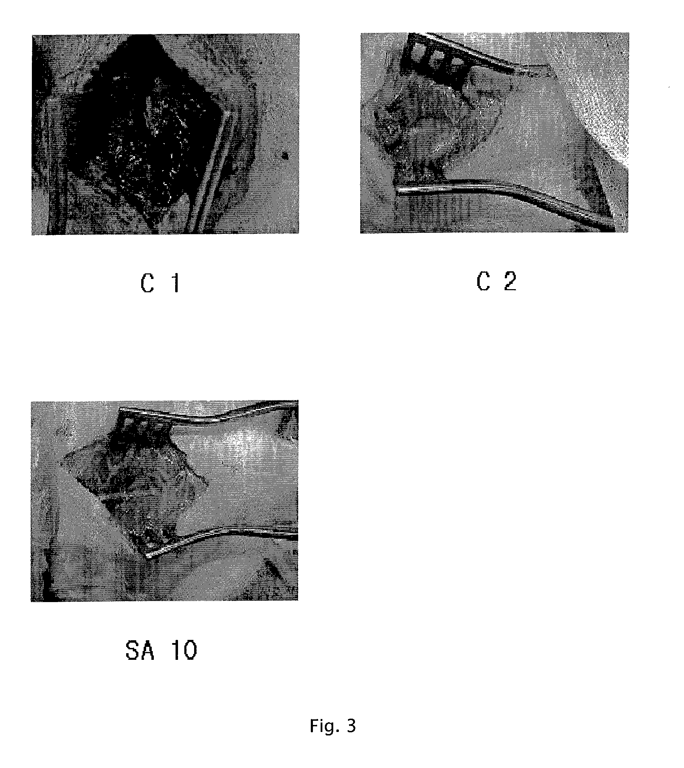

Photographs (see Figure 3) were taken to show the severity of adhesion. In Figure 3, the photograph designated C 1 shows the effect of the comparative gel composition, the photograph designated C 2 shows the effect of Adcon™ and the photograph designated SA 10 shows the effect of gel composition 10. In the sites in which gel compositions 1-1, 1-2 and 2 to 14 had been used, almost similar appearances were identified and photograph SA 10 is representative of all of these.

As may be seen in photograph SA 10, when the anti-adhesion barrier of the invention comprising NaCMC and gellan gum was administered, adhesion at the suture site from the first operation was not found and symptoms such as bleeding or inflammation were not discovered. On the contrary, when typical anti-adhesion barriers known already (comprising only NaCMC and Adcon™, photographs C 1 and C 2 respectively) were administered, adhesion was found at the suture site, as were symptoms such as bleeding and inflammation.

It appears that, when the anti-adhesion barrier contained only NaCMC, this polymer was absorbed into the body so fast that the anti-adhesion effect was lost (Fertil. Steril., 1984, June, 41: 6, 926-928; Fertil. Steril., 1984, June, 41: 6, 929-932; Am. J. Obstet. Gynecol., 1986, 155: 3, 667-670). However, when gellan gum was also included in the anti-adhesion barrier, as in the invention, the absorption of NaCMC into the body was controlled, obviating the disadvantages of NaCMC alone.

Furthermore, the anti-adhesion barrier composition of the invention was verified to have better effect than the commercially available anti-adhesion barrier Adcon™.

### (Experiment 3)

This experiment was conducted using the comparative gel composition and the gel composition 1-1 of the invention.

Mice aged 8 weeks (SD rat, 280 ∼ 400g, male) were opened in the abdominal region and blood vessels situated in between the abdominal skin and organs were cut so that they bled. Then, the comparative gel composition and the gel composition 1-1 were applied and the mice were closed. For each composition, three samples were prepared.

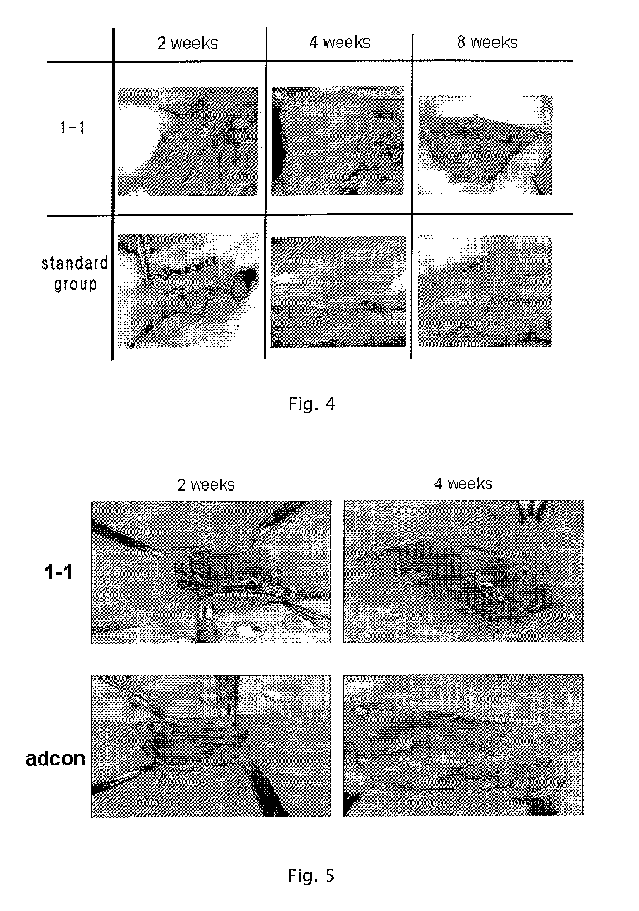

After 2, 4 and 8 weeks from the operation, the operative site was opened and the presence or absence of adhesions was investigated. Photographs were taken, see Figure 4. The photographs designated 1-1 show the effect of the gel composition 1-1 and the photographs designated standard group show the effects of the comparative gel composition. As can be seen in the photographs, when the anti-adhesion barrier of the invention was applied, adhesion between abdominal skin and organ was not observed even after 8 weeks. On the contrary, when the comparative gel composition containing only NaCMC was used, strong adhesion between abdominal skin and organs was identified after 2 weeks. The gellan gum which is included in the composition of the invention was thus verified once more to have preventive effects upon adhesion between tissues, by controlling the absorption of NaCMC into the body and by its lubricating effect.

### (Experiment 4)

This experiment was conducted using Adcon™ and the gel composition 1-1 of the invention.

Mice aged 8 weeks (SD rat, 280 to 400g, male) were opened in the abdominal region and the blood vessels of muscles around the spine were cut so as to have a small injury and bleed. Then, Adcon™ was applied as prescribed and gel composition 1-1 was applied and the mice were closed. For each composition, two samples were prepared.

After 4 and 8 weeks from the operation, the operative site was opened and the presence or absence of bleeding was investigated. Photographs were taken, see Figure 5. The photographs designated 1-1 show the effect of the gel composition 1-1 and the photographs designated adcon show the effects of Adcon™. As can be seen in the photographs, when the anti-adhesion barrier of the invention was applied, adhesion between abdominal skin and organ was not observed even after 4 weeks and also bleeding did not happen during the process of separating tissues. But, in the sample to which Adcon™ was applied, adhesion between skin tissue and muscle tissue was found after 2 weeks to such a degree that bleeding was caused when the tissues were separated.

### Example 5

### In vivo degradation of the anti-adhesion barrier

The degradation over time of the anti-adhesion barrier of the invention was investigated in this experiment.

15 Mice aged 8 weeks (SD rat, 280 ∼ 400g, male) were cut in the abdominal region and opened between the abdominal skin and the panniculus adiposus with about 5 cm of radius. Then, blood vessels of muscles around spine were cut so as to have a small injury and bleed. Afterwards, gel composition 1-1 was applied in 10 ml to a interval between opened abdominal skin and panniculus adiposus and sealed to be recovered forward the original position of skin.

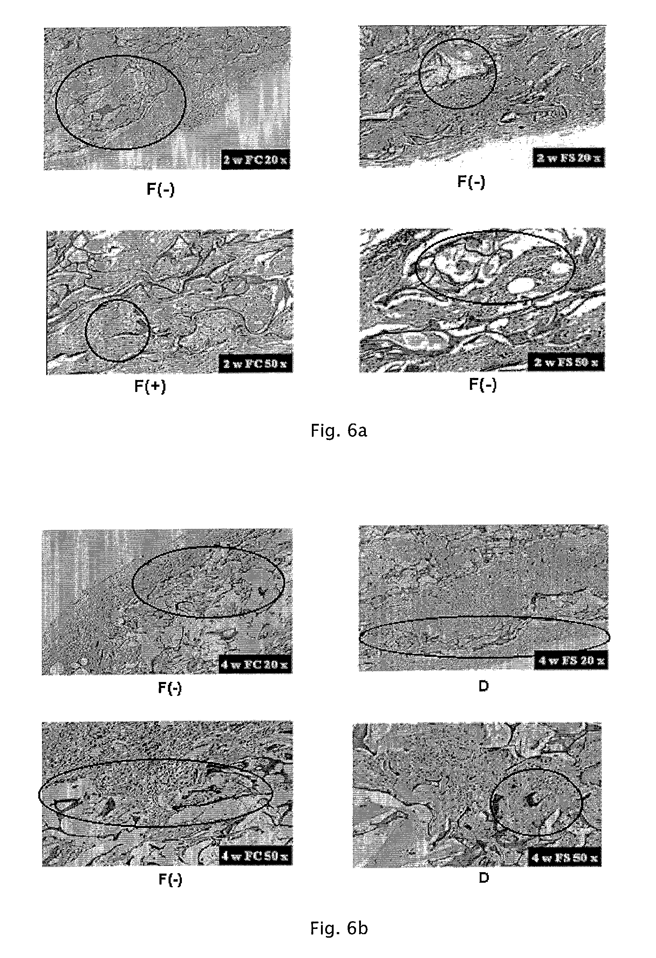

After 2, 4 and 6 weeks from the operation, the operative sites were collected from 5 mice respectively and then treated and stained according to typical sampling methods for animal tissue. The samples were observed under a microscope at 80x and 200x and photographs were taken, see Figures 6a and 6b. Figure 6a depicts the results 2 weeks after administration and Figure 6b the results 4 weeks after administration. In Figures 6a and 6b, the designation F(+) indicates that a fibrosis phenomenon was induced, F(-) that a fibrosis phenomenon was inhibited, D that the anti-adhesion barrier was degraded. Also in Figures 6a and 6b, white colour is indicative of the anti-adhesion barrier and red colour of gel in which the anti-adhesion barrier and secreted blood are conjugated; black dots denote fibrosis and remaining portion are body tissues.

As illustrated in Figures 6a and 6b, adhesion between tissues of surgical lesions abdominal skin was observed to be prevented after 2 and 4 weeks from the operation. Then, after 4 weeks from the operation, most of the anti-adhesion barrier was degraded and absorbed but the adhesion between tissues was identified not to be caused at all. Although not illustrated, after 6 weeks from the operation the anti-adhesion barrier was rarely found.

When the composition of the invention was administered, tissues at a lesion site affected through the operation were not adhered. Moreover, it was confirmed that this can be sustained for a period sufficient for recovery to the original state, whereafter the anti-adhesion barrier is degraded and absorbed..

## Claims

1. An anti-adhesion barrier composition comprising sodium carboxymethyl cellulose and gellan gum in a weight ratio of 1 : (0.2 to 5).

2. A composition according to claim 1 in which the weight ratio of sodium carboxymethyl cellulose to gellan gum is 1 : (0.8 to 1.25).

3. A composition according to claim 1 or claim 2 further comprising from 0.2 to 5 parts by weight of one or more of interleukine-10, hyaluronate, heparin, nifedipine HCl, phosphatidylcholine, medroxyprogestone acetate, L-arginine, nitric oxide, polyvinylalcohol (PVA), protein inhibitor and dextran per 1 part by weight of the sodium carboxymethyl cellulose.

4. A composition according to any preceding claim further comprising from 0.02 to 0.06 parts by weight of an antibiotic substance per 1 part by weight of the sodium carboxymethyl cellulose.

5. A composition according to any preceding claim, which composition is in the form of a powder, foam or gel.

6. A method for the preparation of an anti-adhesion barrier composition according to any of claims 1 to 4 in the form of a foam, the method comprising preparing a solution of the components specified in claim 1 in the ratio specified in claim 1 or claim 2, and optionally the components specified in claim 3 and/or claim 4 in the ratio(s) specified in those claims, in a sufficient amount of water; rapidly freezing the solution by submersing it in a container in liquid nitrogen; and lyophilizing in a reduced pressure at -55°C for more than 48 hours.

7. A method according to claim 6 further comprising the step of autoclaving the solution at from 120 to 150°C for from 5 to 20 minutes before freezing it.

8. A method for the preparation of an anti-adhesion barrier composition according to any of claims 1 to 4 in the form of a powder, the method comprising pulverizing an anti-adhesion barrier composition prepared according to claim 6 or claim 7 to attain a particle radius of from 0.2 to 0.6 mm.

9. A method for the preparation of an anti-adhesion barrier composition according to any of claims 1 to 4 in the form of a gel, the method comprising stirring 1 part by weight of a powder prepared according to claim 8 into from 4 to 19 parts by weight of water.
